**Europäisches Patentamt**

**European Patent Office**

**Office européen des brevets**

(11) Numéro de publication : **0 462 252 B1**

(12) **FASCICULE DE BREVET EUROPEEN**

(45) Date de publication du fascicule du brevet :
26.01.94 Bulletin 94/04

(51) Int. Cl.$^5$ : **C07C 47/21,** C07C 45/71, C07C 45/51

(21) Numéro de dépôt : **91901825.9**

(22) Date de dépôt : **28.12.90**

(86) Numéro de dépôt international :
**PCT/FR90/00958**

(87) Numéro de publication internationale :
**WO 91/09830 11.07.91 Gazette 91/15**

(54) **PROCEDE DE PREPARATION DU CITRAL.**

(30) Priorité : **03.01.90 FR 9000018**

(43) Date de publication de la demande :
**27.12.91 Bulletin 91/52**

(45) Mention de la délivrance du brevet :
**26.01.94 Bulletin 94/04**

(84) Etats contractants désignés :
**AT BE CH DE DK ES FR GB GR IT LI LU NL SE**

(56) Documents cités :
**EP-A- 0 344 043
CH-A- 592 035
DE-A- 2 305 629
CHEMICAL ABSTRACTS, VOL: 83, 1975,
Columbus, Ohio, US; see p.886, summary No.
10500r & JP-A-74133312**

(73) Titulaire : **RHONE-POULENC SANTE
20, avenue Raymond Aron
F-92165 Antony Cédex (FR)**

(72) Inventeur : **CHABARDES, Pierre
24, rue Jeanne-d'Arc
F-69110 Ste-Foy-lès-Lyon (FR)**
Inventeur : **CHAZAL, Jacques
78, avenue Jean-Jaurés
F-69190 S.-Fons (FR)**

(74) Mandataire : **Le Pennec, Magali et al
RHONE-POULENC RORER SA, Direction des
Brevets, 20 Avenue Raymond Aron
F-92165 Antony Cédex (FR)**

## Description

La présente invention concerne un procédé de préparation du citral à partir du prénol et du prénal.

Elle concerne plus particulièrement un procédé perfectionné de préparation de citral à partir de prénol et de prénal.

Il est connu, d'après le brevet français N° 72.40678 publié sous le numéro 2.160.525, de préparer des aldéhydes à liaison $\alpha,\beta$-éthyléniques par réaction à température élevée en phase liquide d'un aldéhyde à liaison $\alpha,\beta$-éthylénique et d'un alcool allylique. L'aldéhyde à liaison $\alpha,\beta$ éthylénique peut être la diméthyl-3,3 acroléine connue sous le nom de prénal, et l'alcool éthylénique peut être le méthyl-3 butène-2 ol-1 connu sous la dénomination de prénol.

La réaction de condensation est réalisée de préférence en présence d'un catalyseur acide à des températures comprises entre 100 et 250°C. L'acide utilisé comme catalyseur est choisi parmi les acides non organiques tels que sulfuriques, phosphoriques, halogénés, nitrique, sulfureux, phosphoreux, perchlorique, borique, silicique, les sels d'acides à hydrogène dissociable, les acides hétérogènes. On peut aussi utiliser les acides organiques. La quantité d'acide utilisée lorsqu'il s'agit d'un acide minéral présentant un PK de 0 à environ 3 est comprise entre 0,01 et 0,5 % au cours de l'ensemble de la réaction qui peut être divisée en deux étapes réactionnelles : formation de l'acétal puis décomposition de ce dernier par crackage.

Il est encore connu d'après le brevet US 4.288.636 de préparer le citral par chauffage d'un acétal diprénylique en présence d'un liquide inerte ayant à la pression de réaction un point d'ébullition supérieur au prénol et inférieur au citral. Le liquide inerte est de préférence le 3,3,7-triméthyl-4 oxa octa-1,6-diène.

La quantité d'acide phosphorique utilisée est comprise entre 0,001 et 0,5 % en poids et de préférence entre 0,005 et 0,05 % en poids.

Il est également connu d'après le brevet FR 2.353.512 de préparer les acétals par condensation d'un aldéhyde sur un alcool en présence d'un acide distillable qui est de préférence l'acide nitrique. Dans ce cas, la quantité d'acide utilisée varie entre $10^{-6}$ % et 1 % en poids par rapport aux composés de départ et de préférence entre $10^{-4}$ et $10^{-2}$ %. Il est même précisé dans ce texte que lorsque l'on utilise un acide moins fort, par exemple l'acide phosphorique, la quantité d'acide peut aller jusqu'à 10 % c'est-à-dire est multipliée par dix par rapport à l'acide nitrique.

Rien n'indique, même en rassemblant ces trois documents dont certains et particulièrement le troisième ont des zones de concentration très larges, que les concentrations en acide doivent être différentes dans les deux étapes : acétalisation et crackage puisque les plages indiquées de concentration ont une certaine zone de recouvrement.

En comparant ces trois textes, il semble même que l'acidité nécessaire pour l'acétalisation serait inférieure ou égale à l'acidité nécessaire pour le crackage.

En mettant en oeuvre ces procédés au niveau du laboratoire, nous nous sommes aperçus que l'acidité nécessaire pour la formation de l'acétal et l'acidité nécessaire pour l'étape de crackage étaient différentes et que le rendement était amélioré en maîtrisant soigneusement l'acidité du milieu réactionnel au cours du procédé.

La présente invention concerne donc un procédé amélioré de préparation du citral à partir du prénol et du prénal caractérisé en ce que dans la même enceinte réactionnelle on effectue :

- dans une première étape la condensation du prénol avec le prénal en présence d'un acide minéral présentant une concentration d'environ $5.10^{-3}$ mole par mole de prénal introduite et d'un solvant formant un azéotrope avec l'eau,
- dans une deuxième étape, on neutralise 90 % à 95 % de l'acidité,
- dans une troisième étape, on chauffe le milieu réactionnel, on élimine le prénol et le prénal qui sont recyclés à la première étape puis on distille le citral.

L'utilisation d'une acidité très faible au cours de l'étape de crackage permet d'augmenter les rendements en citral.

Ainsi, lorsque lors de l'étape de crackage on utilise une concentration en acide de $10^{-3}$ mole par mole d'acétal (correspondant à 1 mole de prénal d'origine) on n'obtient pas de citral (exemple 2). Par contre, lorsque lors de l'étape d'acétalisation on utilise une concentration en acide de $10^{-4}$ mole par mole de prénal, le rendement d'acétalisation atteint au maximum 30 % (exemple 3).

Il n'était pas évident à la lecture des brevets précédemment cités d'en conclure que la réaction d'acétalisation devait être réalisée avec une concentration d'acide cinq à dix fois plus importante que la réaction de crackage pour que le procédé puisse être mis en oeuvre de façon économiquement rentable.

L'acide utilisé est de préférence l'acide phosphorique.

Le rapport molaire du prénol calculé par rapport au prénal est de préférence compris entre 2 et 2,5.

Le solvant formant azéotrope avec l'eau est de préférence le toluène.

Il peut aussi être avantageux d'ajouter des traces d'hydroquinone.

La réaction d'acétalisation est réalisée de préférence à une température comprise entre 60 et 90°C sous une pression comprise entre 8 et $14.10^3$ pascals (6 à 10 cm de mercure).

Le toluène est distillé avec l'eau formée en cours de réaction.

La neutralisation de l'acidité est réalisée au moyen d'une base choisie parmi les sels alcalins de l'acide acétique de préférence l'acétate de potassium, l'acétate de sodium, ainsi que parmi les carbonates acide de sodium ou de potassium ou les hydroxydes de métaux alcalins .

On neutralise entre 90 % et 95 % de l'acide introduit.

Le crackage de l'acétal formé est réalisé ensuite sur le brut de réaction sans élimination des produits secondaires de condensation et sans isoler l'acétal diprénylique en dégageant cependant l'excès de prénal et de prénol introduits au départ.

Le crackage de l'acétal est réalisé à une température comprise entre 120 et 150°C sous une pression comprise entre 8 et $16.10^3$ pascals (6 et 12 cm de mercure). On élimine d'abord le prénol formé lors de la réaction de crackage puis on chauffe le milieu à une température de 120°C pour distiller le citral.

La présente invention sera plus complètement décrite à l'aide des exemples suivants qui ne doivent en aucun cas être considérés comme limitatifs de l'invention.

## EXEMPLE 1

### Acétalisation

Dans un réacteur, on introduit :
- 35,1 g de prénal à 97,2 % (0,405 mole)
- 87,0 g de prénol à 100 % (1,010 mole)
- 10,0 g de toluène
- 2 ml d'acide phosphorique en solution 0,98 molaire contenant 1,960 mmoles d'acide phosphorique.

On utilise une colonne de distillation contenant 5 plateaux théoriques. On chauffe le milieu réactionnel pendant 6 heures à une température comprise entre 72 et 90°C à une pression de 80 mm de mercure. On distille 8,5 ml d'eau. Après réaction, on distille le toluène en un quart d'heure. On augmente le vide jusqu'à 15 mm de mercure pour soutirer le reste du toluène.

Le taux de transformation du prénal qui est le rapport :

$$\frac{(\text{prénal introduit} - \text{prénal récupéré})}{\text{prénal introduit}}$$

est de 83 %, le rendement en acétal diprénylique qui correspond au rapport :

$$\frac{(\text{acétal diprénylique formé})}{\text{prénal introduit}}$$

est de 75,6 %.

### Neutralisation

On neutralise le milieu réactionnel en ajoutant 180 mg d'acétate de potassium et 100,5 mg d'hydroquinone.

La solution contient alors $4.10^{-4}$ mole d'acide phosphorique.

On réalise alors une distillation sous 15 mm de mercure de façon à récupérer le prénal et le prénol excédentaires (31,8 g de récupéré).

### Crackage

On chauffe ensuite le milieu réactionnel 3 heures 30 à 125°C-140°C sous 90 mm de mercure. On distille le prénol formé en cours de réaction puis le citral en laissant la température à 120°C et en baissant la pression jusqu'à 0,05 mm de mercure. On récupère 45,8 g de distillat contenant 38,9 g de citral ce qui représente un rendement de 63 % par rapport au prénal engagé et de 78 % par rapport au prénal transformé.

## EXEMPLE 2 (COMPARATIF)

### Acétalisation

On introduit :
- 33,6 g de prénal à 99,5 % (397 mmoles)
- 86 g de prénol à 99,6 % (994 mmoles)
- 200 μl d'acide phosphorique à 85 % correspondant à (2,95 mmoles) : 0,17 % en poids : $7,4 \ 10^{-3}$ mole/mole prénal

Après réaction dans les mêmes conditions qu'à l'exemple 1, on obtient une masse réactionnelle de 111,9 g dans laquelle on dose le prénol, le prénal et l'acétal formé. Les taux de conversion sont de 77% pour le prénal et 64% pour le prénol. Le rendement en acétal par rapport au prénal chargé est de 74%.

On n'effectue pas de neutralisation, on vérifie par dosage potentiométrique que tout l'acide phosphorique chargé initialement est présent dans le brut réactionnel. On ajoute de l'hydroquinone sur ce mélange qui contient l'acétal, l'hydroquinone et l'acide phosphorique dans un rapport molaire $1/23.10^{-4}/100.10^{-4}$.

### Crackage

On effectue le crackage dans les mêmes conditions qu'à l'exemple 1 en chauffant à une température de 97°C pour ne pas dégrader l'acétal mais en baissant la pression à 15 mm de mercure. On n'obtient pas de traces de citral mais seulement des polymères, des produits lourds et des éthers.

## EXEMPLE 3 (COMPARATIF)

### Acétalisation

On introduit :
- 25,2 g de prénal à 99,5 % (298 mmoles)
- 64,6 g de prénol à 99,6 % (747 mmoles)
- 25,2 g de cyclohexane
- 140 mg d'acide phosphorique à 0,98 M (0,137 mmole) soit 4,6 10⁻⁴ mole par mole de prénal.

On chauffe à 110°C à la pression atmosphérique 3 heures puis sous 250 mm de mercure pendant 2 heures.

Le taux de transformation du prénal n'est que de 28 %. Par contre l'addition d'acide pour porter sa concentration relative au prénal aux mêmes valeurs que celles utilisées dans les exemples précédents permet de poursuivre la réaction.

## Revendications

1. Procédé de préparation de citral à partir de prénal et de prénol caractérisé en ce que dans la même enceinte réactionnelle on effectue en phase liquide

   . dans une première étape la condensation du prénal avec le prénol en présence d'un solvant formant un azéotrope avec l'eau et d'un acide minéral à une concentration d'environ $5.10^{-3}$ mole pour une mole de prénal

   . dans une deuxième étape on neutralise 90 à 95 % de l'acide minéral introduit

   . dans une troisième étape, après élimination du prénal et du prénol n'ayant pas réagi, on distille le citral.

2. Procédé selon la revendication 1 caractérisé en ce que le solvant de la première étape est le toluène.

3. Procédé selon la revendication 1 caractérisé en ce que l'acide minéral de la première étape est l'acide phosphorique.

4. Procédé selon la revendication 1 caractérisé en ce que l'agent neutralisant est un acétate alcalin de préférence l'acétate de potassium.

## Claims

1. Process for the preparation of citral from prenal and from prenol, characterized in that, in one and the same reaction chamber, there are performed, in the liquid phase,

   in a first stage, the condensation of prenal with prenol in the presence of a solvent which forms an azeotrope with water and of an inorganic acid at a concentration of about $5.10^{-3}$ mol per mol of prenal,

   in a second stage, the neutralization of 90 to 95% of the inorganic acid introduced, and

   in a third stage, distillation of the citral after removal of the unreacted prenal and prenol.

2. Process according to Claim 1, characterized in that the solvent in the first stage is toluene.

3. Process according to Claim 1, characterized in that the inorganic acid in the first stage is phosphoric acid.

4. Process according to Claim 1, characterized in that the neutralizing agent is an alkali metal acetate, preferably potassium acetate.

## Patentansprüche

1. Verfahren zur Herstellung von Citral aus Prenal und Prenol, dadurch gekennzeichnet, daß man in demselben Reaktionsgefäß in flüssiger Phase bewirkt:

   - in einer ersten Stufe die Kondensation des Prenals mit dem Prenol in Gegenwart eines Lösungsmittels, das mit Wasser ein Azeotrop bildet und einer Mineralsäure mit einer Konzentration von etwa $5 \times 10^{-3}$ Mol für ein Mol Prenal,

   - in einer zweiten Stufe 90 bis 95 % der eingeführten Mineralsäure neutralisiert,

   - in einer dritten Stufe nach Entfernung des Prenals und des Prenols, die nicht reagiert haben, das Citral abdestilliert.

2. Verfahren gemäß Anspruch 1, dadurch gekennzeichnet, daß das Lösungsmittel der ersten Stufe Toluol ist.

3. Verfahren gemäß Anspruch 1, dadurch gekennzeichnet, daß die Mineralsäure der ersten Stufe Phosphorsäure ist.

4. Verfahren gemäß Anspruch 1, dadurch gekennzeichnet, daß das Neutralisationsmittel ein Alkaliacetat, vorzugsweise Kaliumacetat ist.